# EUROPEAN PATENT APPLICATION

(11) **EP 1 623 663 A1**
(43) Date of publication of application: **08.02.2006**
(21) Application number: 04732475.1
(22) Date of filing: 12.05.2004
(51) Int. Cl.: A61B 1/00

(54) **ELECTRICALLY BENDING ENDOSCOPE**

(30) Priority: 14.05.2003 JP 2003136393
(71) Applicant: Olympus Corporation, Tokyo 151-0072 (JP)
(72) Inventor: MAEDA, Toshinari, c/o Olympus Corporation, Shibuya-ku, Tokyo 151-0072 (JP); ARAI, Keiichi, . (JP); IKEDA, Yuichi, . (JP); MIYAGI, Takayasu, . (JP)
(74) Representative: von Hellfeld, Axel
(86) International application number: PCT/JP2004/006671
(87) International publication number: WO 2004/100775

(57) **Abstract**

An electrical bending endoscope includes: a driving-force transmitting mechanism that transmits the driving force for bending a bending portion (12b); a motor that supplies the driving force to the driving-force transmitting mechanism; a driving-force interrupting mechanism that is arranged to a transmitting line of the driving force transmitted from the motor to the bending portion (12b) and interrupts the transmission of the driving force; and a switching operating member (23x) that operates the interrupt of the driving-force interrupting mechanism. The electrical bending endoscope further includes a click mechanism including a click spring (24) that generates the inconstant amount of change in force upon operating the switching operating member (23x) and click concave portions (22d and 22e) and the state of the switching operating member (23x) is sensed by an operator, thereby preventing the erroneous operation.

## Description

### Technical Field

The present invention relates to an electrical bending endoscope, and more particularly, to an electrical bending endoscope having a bending mechanism which can bend a bending portion by using electrical driving force, and a driving-force interrupting mechanism (clutch mechanism) arranged to a driving-force transmitting mechanism, for interrupting the transmission of the driving force.

### Background Art

For example, Japanese Unexamined Patent Application Publication No. 5-95896 discloses one of various conventional electrical bending endoscopes, wherein a driving member including an electrical motor and the like in an endoscope control section and a bending portion of an endoscope insertion section is remotely bent by using the driving force generated by a driving member.

Generally, in the conventional electrical bending endoscopes, a driving-force transmitting mechanism including a traction member (wire) comes between a driving member (driving portion), such as a driving motor, and a bending portion (driven portion), so that the driving force in the rotating direction generated by the driving member can be converted into the driving force in the desired direction, and the driving force can be transmitted to the bending portion. Thus, the rotating driving force generated by the driving member (driving motor) arranged on the side of a control section is transmitted to the bending portion via the driving-force transmitting mechanism. As a consequence, the bending portion can be freely bent within a predetermined range.

The entire operation of the conventional electrical bending endoscopes depends on the driving force of the driving member, thereby always connecting the driving member, the driving-force transmitting mechanism, and the bending portion. In this state, for example, when the power supplied to the driving member stops, the bending portion stops with the bending state of the bending portion being fixed, depending on the rotating stop position of the driving member. Even in the state, the driving member always tracts the traction member (wire), thereby continuously applying the large load to the traction member.

That is, in the endoscopy using the electrical bending endoscope, the power is supplied to the driving member, thereby rotating the driving member. In this case, the driving member, the driving-force transmitting mechanism, and the bending portion are connected so that the driving force of the driving member is always transmitted to the bending portion via the driving-force transmitting mechanism.

On the other hand, when the power supply to the driving member stops, e.g., upon conveying or keeping the endoscope, that is, at the time with the exception of the time using the endoscopy, it is necessary that the amount of driving force of the driving member does not reach the bending portion.

Thus, in the driving-force transmitting mechanism of the conventional electrical bending endoscopes, generally, a driving-force interrupting mechanism (hereinafter, referred to as a clutch mechanism) is arranged on a transmitting line for transmitting the driving force from the driving member to the bending portion. The driving-force interrupting mechanism arbitrarily switches the state for transmitting the driving force generated by the driving member to the bending portion and the state for transmitting no driving force, that is, the state for interrupting the transmitting line of the driving force. The driving-force interrupting mechanism is arranged between a rotation driving shaft of the driving member and a predetermined component for transmitting the driving force from the rotation driving shat to the traction member.

The arrangement of the clutch mechanism enables arbitrary interrupt of the connection between the driving member and the driving-force transmitting mechanism. Upon releasing the connection between the driving member and the driving-force transmitting mechanism (that is, in the interrupting state), the amount of force of the driving member does not reach the driving-force transmitting mechanism and therefore the load is not applied to the bending portion. Hence, the fixing state with unnatural bending state is prevented.

As mentioned in Japanese Unexamined Patent Application Publication No. 5-95896, the electrical bending endoscope comprises the control section including a main frame, serving as a fixing member of the control section and a motor frame that is freely slid and moved to the main frame and fixedly mounts a driving motor for rotating a driving gear. The motor frame comprises a locking member that keeps the engaged state between a driving gear that is axially supported to a rotating shaft of the driving motor and a driving gear that is connected to a bending wire. A predetermined operating member (cam plate) is subjected to predetermined locking operation, thereby locking the locking member to the fixing member on the main frame side. The operating member (cam plate) is subjected to predetermined release operation, thereby releasing the locking state of the locking member. Thereafter, the motor frame is slid and moved to the main frame and then both the gears in the engaged state are separated from each other.

As mentioned above, the predetermined operating member is arbitrarily operated, thereby arbitrarily interrupting the driving force on the transmitting line of the driving force transmitted from the driving member to the bending portion.

Generally, the switching operating member of the above-mentioned clutch mechanism is arranged at a predetermined position of the control section. Thus, upon using the endoscope, the amount of unintentional external force to the switching operating member is prevented. Even if the amount of force is applied, the switching operation must not easily be performed. Further, a measure for preventing the erroneous operation of a user must be taken. When the user intends, the disconnection and connection of the clutch mechanism must be executed without fail.

The present invention is devised in consideration of the above-mentioned points. One object of the present invention is to provide an electrical bending endoscope, in which the operation of the clutch mechanism applied to the electrical bending endoscope enables the switching of disconnection and connection of the driving-force transmitting line without fail, and the operability is improved and the safety is ensured by certainly keeping the setting state and notifying the switching to the user's setting state in the switching operation.

Further, the other object of the present invention is to provide an electrical bending endoscope, in which the switching operation is not easily executed by the amount of external force to the switching operating member and the higher safety is ensured.

### Disclosure of Invention

According to the first aspect of the present invention, an electrical bending endoscope comprises: driving means, serving as a supply source of driving force; driving-force transmitting means that transmits the driving-force supplied from the driving means to a bending portion to be bent; driving-force interrupting means that is arranged to a transmitting line of the driving-force transmitted from the driving means to the bending portion and interrupts the transmission of the driving force; switching operating means that is interlocked with the driving-force interrupting means, to interrupt the transmission of the driving force via the driving-force interrupting means upon its operation; and error-operation preventing means that prevents unintentional operation of the switching operating means.

According to the second aspect of the present invention, an electrical bending endoscope comprises: an elongated insertion section having a bending portion that is freely bendable within a predetermined range; a control section that is arranged to the proximal end of the insertion section; a driving source that generates the driving force for bending the bending portion; a driving-force transmitting mechanism that transmits the driving force supplied from the driving source to the bending portion; a driving-force interrupting mechanism that is arranged to a transmitting line of the driving force transmitted from the driving source to the bending portion and interrupts the transmission of the driving force; a switching operating member that is arranged to the control section and mechanically connected to the driving-force interrupting mechanism, to interrupt the transmission of the driving force via the driving-force interrupting mechanism upon its operation; and an error-operation preventing device that prevents unintentional operation of the switching operating member.

### Brief Description of the Drawings

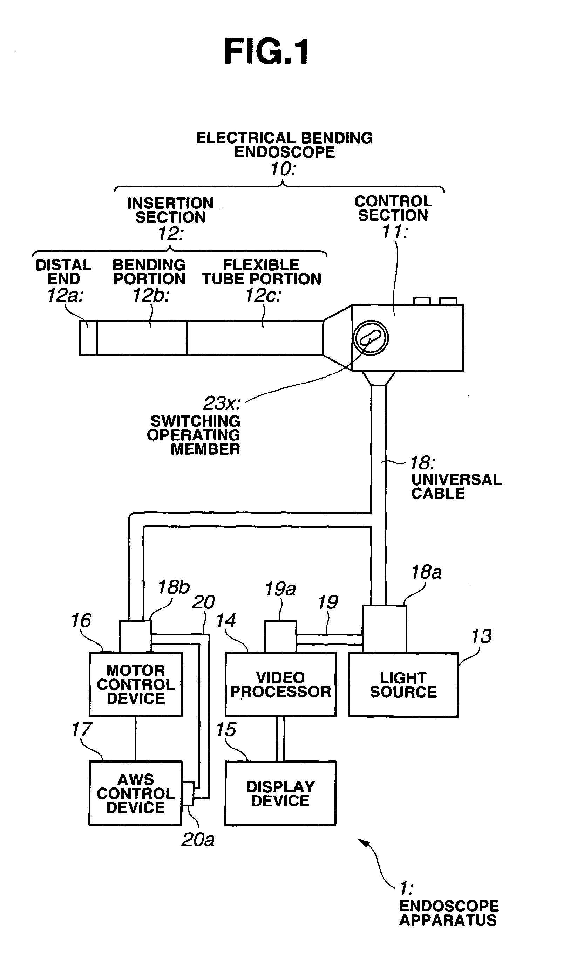

Fig. 1 is a block diagram schematically showing the entire configuration of an endoscope apparatus using an electrical bending endoscope according to a first embodiment of the present invention;

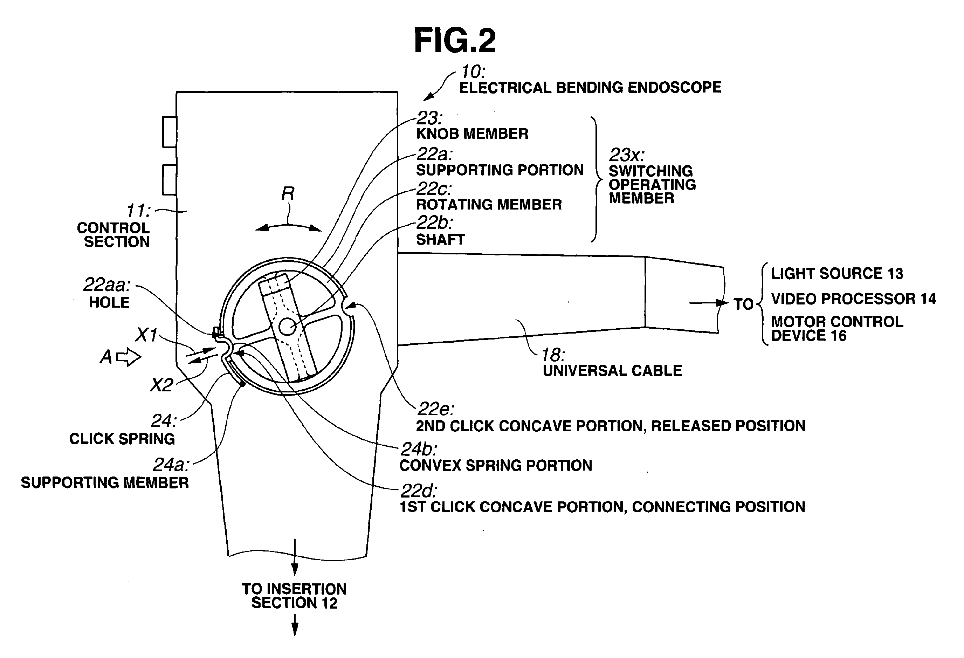

Fig. 2 is a schematic diagram enlarging and showing a part of the configuration of a control section in an electrical bending endoscope, specifically showing the internal configuration in and around a switching operating member of a driving-force interrupting switching mechanism (clutch mechanism) and the state in which the switching operating member is at the connecting position according to the first embodiment of the present invention;

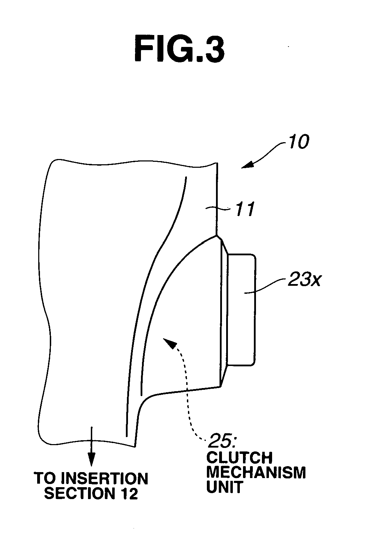

Fig. 3 is a side view of the main portion, being cut out and seen in the direction of an arrow A in Fig. 2, in and around the switching operating member, serving as a part of the control section shown in Fig. 2;

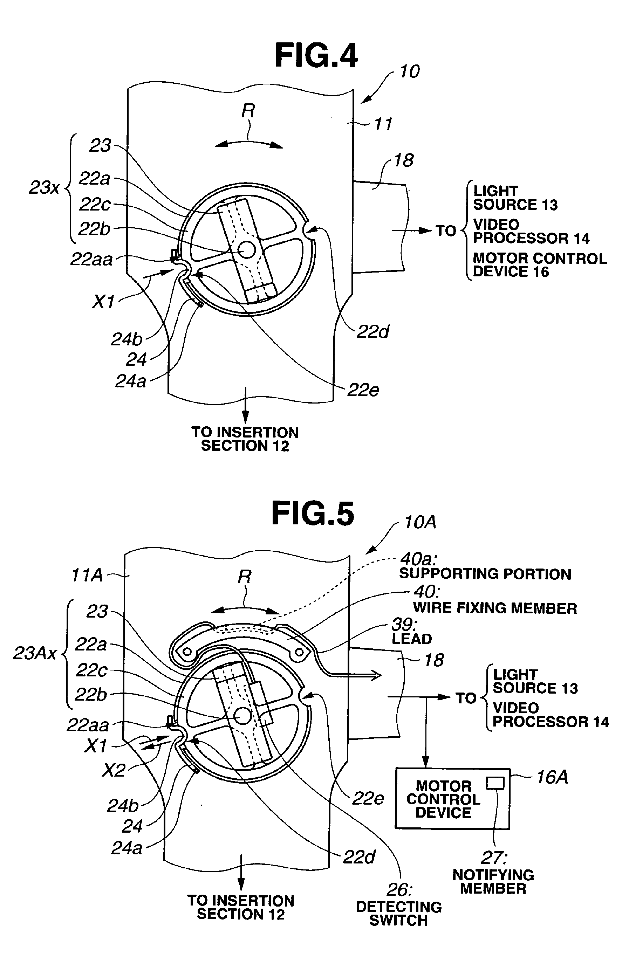

Fig. 4 is a schematic diagram enlarging and showing a part of the control section in the electrical bending endoscope, specifically showing the internal configuration in and around the switching operating member of the driving-force interrupting switching mechanism (clutch mechanism) in which the switching operating member is at a released position according to the first embodiment of the present invention;
Fig. 5 is a schematic diagram enlarging and showing a part of a control section of an electrical bending endoscope according to the second embodiment of the present invention, specifically showing the internal configuration in and around a switching operating member of the driving-force interrupting switching mechanism (clutch mechanism);

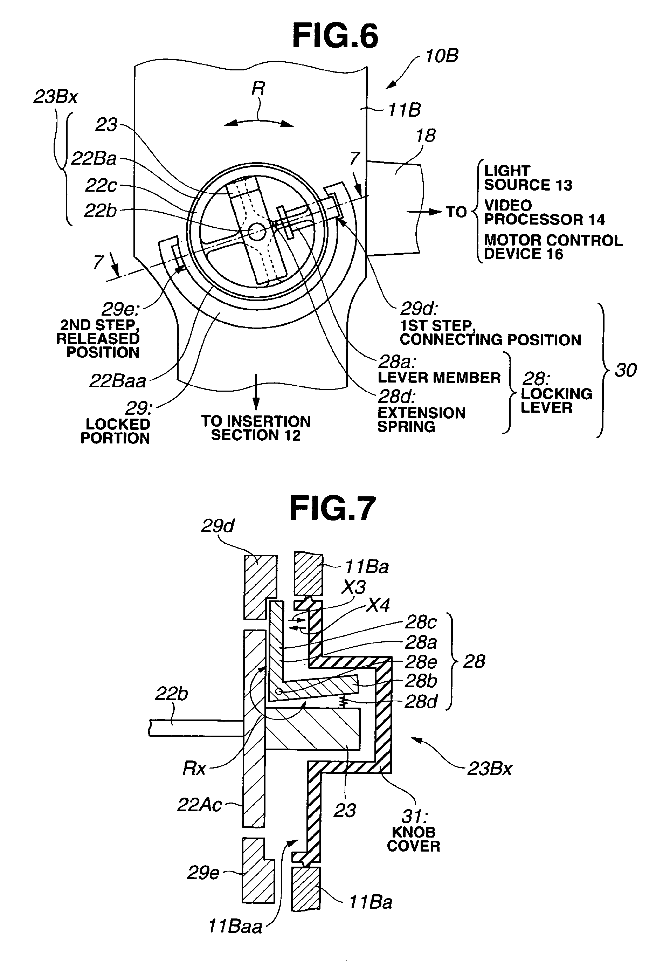

Fig. 6 is a schematic diagram enlarging and showing a part of a control section of an electrical bending endoscope according to the third embodiment of the present invention, specifically showing the internal configuration in and around a switching operating member of the driving-force interrupting switching mechanism (clutch mechanism);
Fig. 7 is a sectional view along a line 7-7 shown in Fig. 6, specifically showing the details of the switching operating member;

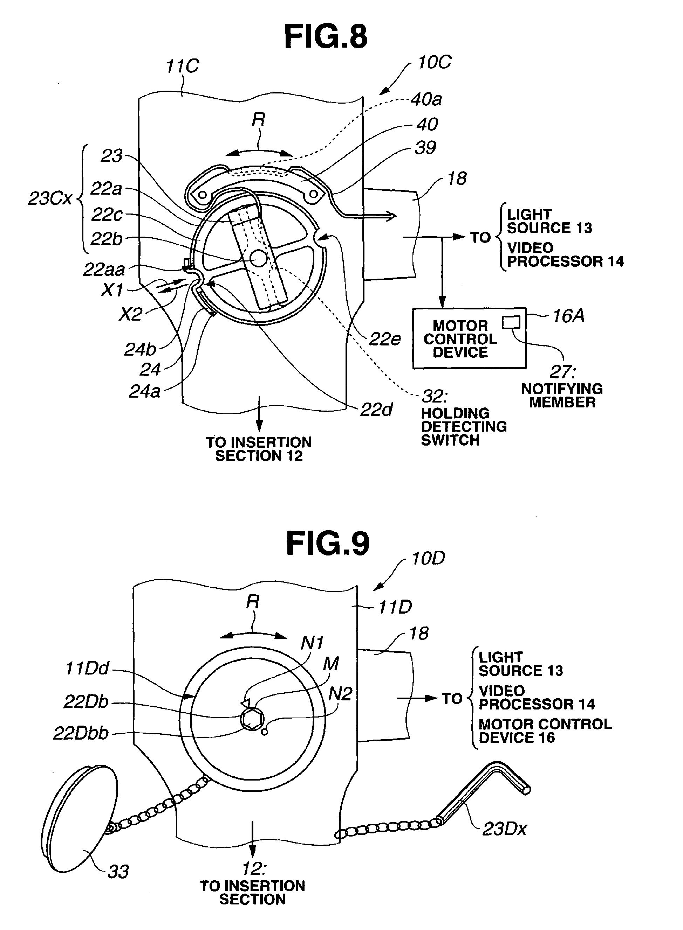

Fig. 8 is a schematic diagram enlarging and showing a part of a control section of an electrical bending endoscope driving-force interrupting switching mechanism (clutch mechanism), specifically showing the internal configuration in and around a switching operating member according to the fourth embodiment of the present invention;
Fig. 9 is a schematic diagram enlarging and showing a part of a control section of an electrical bending endoscope driving-force interrupting switching mechanism (clutch mechanism), specifically mainly showing a switching operating member according to the fifth embodiment of the present invention;

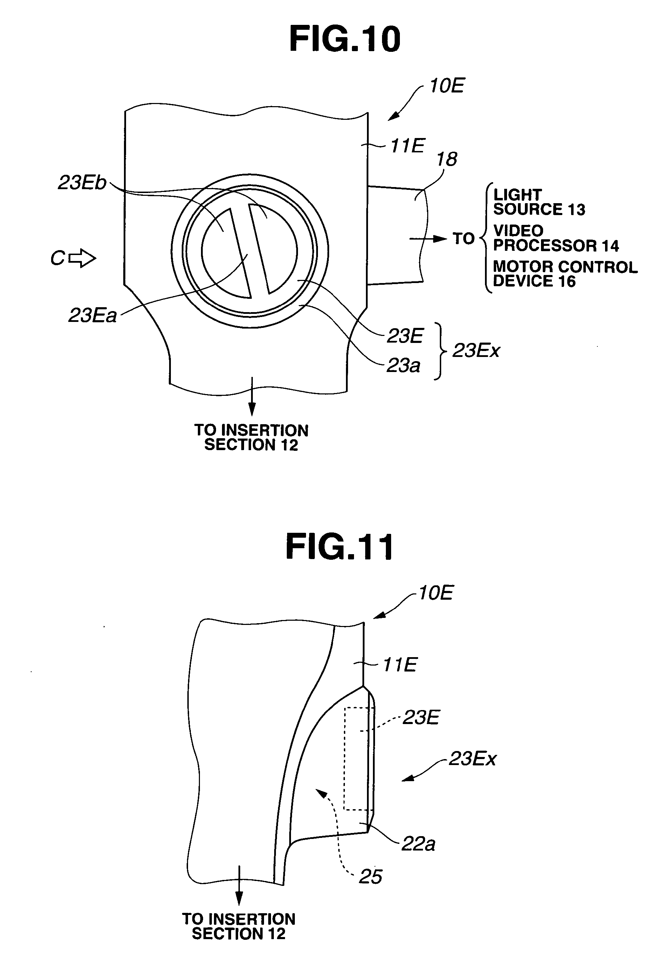

Fig. 10 is a schematic diagram enlarging and showing a part of a control section of an electrical bending endoscope driving-force interrupting switching mechanism (clutch mechanism), specifically showing the configuration in and around a switching operating member according to the sixth embodiment of the present invention;
Fig. 11 is a side view of the main portion, being cut out and seen in the direction of an arrow C shown in Fig. 10, in and around the switching operating member, serving as a part of the control section shown in Fig. 10;

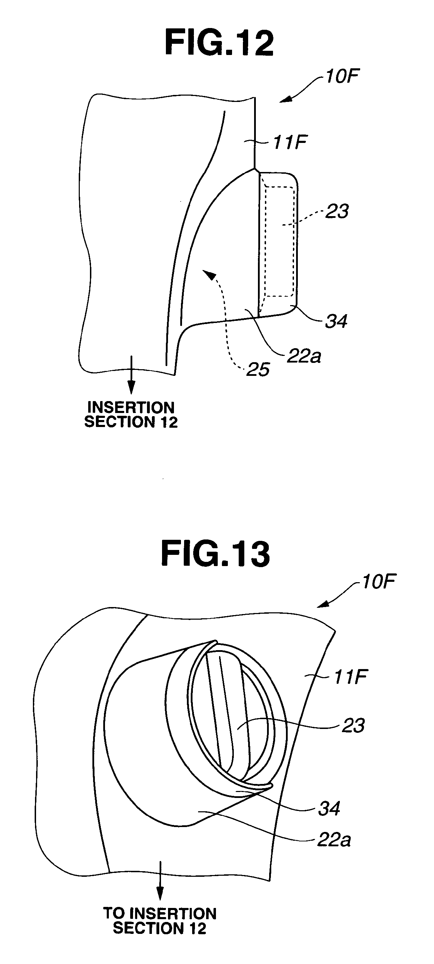

Fig. 12 is a cut-out side view in and around a switching operating member, serving as a part of a control section in an electrical bending endoscope according to the seventh embodiment of the present invention;
Fig. 13 is a schematic perspective view showing, being cut out and seen from the left of the switching operating member, in and around the switching operating member, serving as a part of the control section in the electrical bending endoscope according to the seventh embodiment of the present invention;

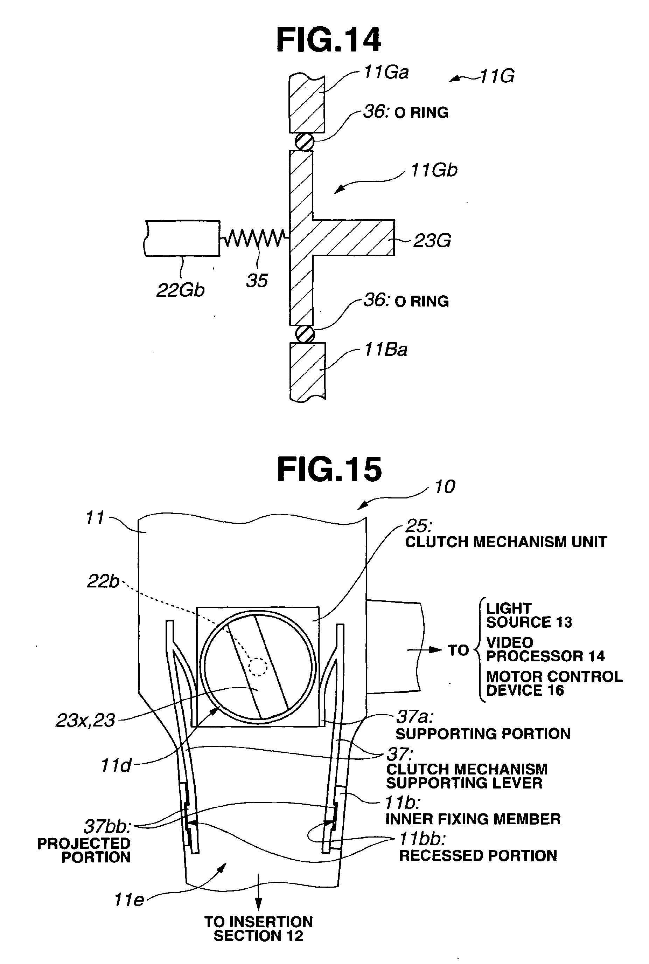

Fig. 14 is a sectional view enlarging and showing a part of a control section in an electrical bending endoscope, specifically schematically showing the internal configuration in and around switching operating member of a driving force interrupting switching mechanism (clutch mechanism) according to the eighth embodiment of the present invention;
Fig. 15 is a schematic diagram enlarging and showing the configuration of a part of a control section in an electrical bending endoscope, specifically showing the internal configuration in and around switching operating member of a driving-force interrupting switching mechanism (clutch mechanism); and

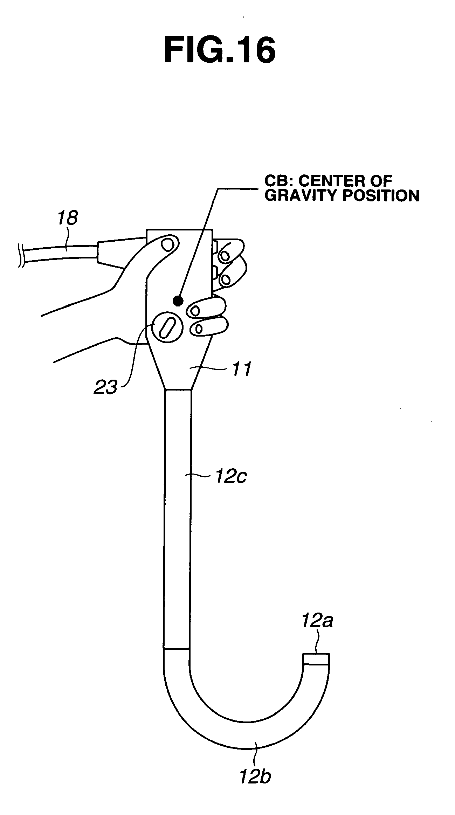

Fig. 16 is a diagram showing a center of gravity position CB of the control section of the electrical bending endoscope according to the embodiments.

### Best Mode for Carrying Out the Invention

Hereinbelow, embodiments of the present invention will be described with reference to the drawings.

First, before specifically describing an electrical bending endoscope according to the first embodiment, a description is given of the entire schematic configuration of an endoscope apparatus using an electrical bending endoscope with reference to Fig. 1.

Fig. 1 is a block diagram schematically showing the entire configuration of the endoscope apparatus using an electrical bending endoscope according to a first embodiment of the present invention.

Referring to Fig. 1, an endoscope apparatus 1 comprises: an electrical bending endoscope (hereinafter, simply abbreviated to an endoscope as needed) 10; a light source 13 that supplies the illumination luminous flux to the endoscope 10; a video processor 14 that receives a signal outputted from the endoscope 10 and performs various predetermined signal processing; a display device 15 that receives a video signal in a predetermined format, outputted from the video processor 14 and displays a predetermined video image based on the video signal; a motor control unit (also referred to as an MCU) 16, serving as a driving control device, for controlling the bending operation of the endoscope 10; and an AWS control device 17 that controls the operation including the air feed, water feed, and absorption via the endoscope 10.

The endoscope 10 comprises: an elongated insertion section 12 that is inserted in the body cavity; a control section 11 arranged to the proximal end of the insertion section 12, comprising various operating members including a switching operating member 23x, serving as switching operating means, for switching the interrupting state of a driving-force interrupting mechanism (clutch mechanism), which will be described later; and a universal cable 18 that is extended from the side portion of the control section 11 and is branched to two lines at the middle point of extended portion from the side portion.

At one end of the universal cable 18, a connector 18a freely detachably connected to the light source 13 is arranged. At the other end of the universal cable 18, a connector 18b freely detachably connected to a motor control device 16 is arranged. Thus, the control section 11 of the endoscope 10, the light source 13, and the motor control device 16 are connected.

From the connector 18a, a cable 19 having a connector 19a at the end thereof is extended. The connector 19a is connected to a corresponding connecting portion of the video processor 14. Thus, the video processor 14 and the control section 11 are connected. Further, the display device 15 is connected to the video processor 14 via a predetermined signal cable 19b.

From the connector 18b of the universal cable 18, a cable 20 having a connector 20a at the end thereof is extended. The connector 20a is connected to a corresponding connecting portion of the AWS control device 17. Thus, the AWS control device 17 and the control section 11 are connected.

The insertion section 12 comprises: a rigid distal end 12a; a bending portion 12b that is continuously arranged to the side of the back end of the distal end 12a and is freely bendable within a predetermined range in the up/down direction and the right/left direction and in the complex direction thereof; and a long flexible portion 12c that is continuously arranged to the side of the back end of the bending portion 12b with flexibility. The side of the back end of the flexible portion 12c is integrally arranged at a predetermined position of the control section 11.

The control section 11 has, on the side of the external surface thereof, a plurality of operating members that perform various operations, e.g., switching operating member 23x, at predetermined positions, and further has therein a driving motor (not shown), serving as a driving member as driving means and a driving source, a driving-force transmitting mechanism (not shown), serving as driving-force transmitting means, which transmits the driving force generated by the driving motor to the bending portion 12b, and a driving-force interrupting mechanism (hereinafter, referred to as clutch mechanism, not shown), serving as driving-force interrupting means, which partly forms the driving-force transmitting mechanism and arbitrarily mechanically switches the interrupt of the transmission of driving force.

Incidentally, the specific configuration of another unit does not directly relate to the essential of the present invention and therefore is the same as that of the conventional electrical bending endoscope. A specific description thereof is omitted and only portions of the features of the present invention will be described in detail.

Figs. 2 and 4 are schematic diagrams enlarging and showing a part of the configuration of a control section in the electrical bending endoscope, specifically showing the internal configuration in and around the switching operating member of the driving-force interrupting switching mechanism (clutch mechanism) and the state in which the switching operating member is at the connecting position according to the first embodiment of the present invention. Incidentally, Fig. 2 shows the state in which the switching operating member is at the connecting position, and Fig. 4 shows the state in which the switching operating member is at the released position. Then, Fig. 3 is a side view of the main portion, being cut out and seen in the direction of an arrow A in Fig. 2, in and around the switching operating member, serving as a part of the control section shown in Fig. 2.

As mentioned above, the control section 11 has a driving-force transmitting mechanism (driving force transmitting means; not shown) including the clutch mechanism. The clutch mechanism is operated by operating a switching operating member 23x (switching operating means), serving as one of operating members, arranged to the side portion of the control section 11.

Referring to Fig. 2, the switching operating member 23x is at a predetermined portion on the side of the control section 11, and is rotatably arranged in the inner space formed inside a supporting portion 22a integrally formed to an inner fixing member (not shown) thereof.

The switching operating member 23x is operated by pinching with the operator's fingers or by another means, and comprises: a knob member 23 that is arranged at the externally-exposing position of the control section 11; a shaft 22b that axially supports freely rotatably the knob member 23 and moves together with a main mechanism portion (not shown) of the clutch mechanism; a rotating member 22c that is assembled to be integrated with the knob member 23 and the shaft 22b; a click spring 24 comprising an elastic member such as a plate spring, and regulating the positioning of the rotating member 22c at a predetermined position; and a supporting member 24a that axially supports one end of the click spring 24.

The rotating member 22c is rotatably arranged along the inner circumference of the supporting portion 22a, and the click spring 24 is arranged at a predetermined position of the supporting portion 22a.

One end of the click spring 24 is fixed to an inner fixing member (not shown) of the control section 11 at a supporting member 24a. At a free end of the other end of the click spring 24, a convex spring portion 24b is formed. The convex spring portion 24b is arranged such that the projected portion thereof is directed to the side of the supporting portion 22a (the rotating member 22c). Corresponding to this, a hole portion 22aa for engaging the convex spring portion 24b of the click spring 24 is pierced at a predetermined position on the outer circumference of the supporting portion 22a.

The convex spring portion 24b is fit into the hole portion 22aa and is arranged such that projected portion of the convex spring portion 24b is slightly projected to the inside of the supporting portion 22a. In order to keep the state, repulsing force of the click spring 24 in the general state operates in the direction of an arrow X1 shown in Fig. 2. Therefore, the convex spring portion 24b always comes into contact with the outer circumference of the rotating member 22c via the hole portion 22aa, and presses the rotating member 22c with predetermined repulsing force.

In other words, the repulsing force of the click spring 24 operates toward the outer circumference of the rotating member 22c with the amount of repulsing force in the operating direction of the clutch mechanism or the switching operating member 23x, that is, in the direction (shown by an arrow X1 in Fig. 2) different from a vector of the rotating direction (shown by an arrow R in Fig. 2).

As mentioned above, the rotating member 22c is assembled to be integrated to the knob member 23 and the shaft 22b. The operator operates the knob member 23, thereby rotating the rotating member 22c in the direction shown by the arrow R in Fig. 2. Then, the rotating member 22c and the shaft 22b are rotated together therewith in the same direction.

On the outer circumference of the rotating member 22c, two click recessed portions, that is, a first-click concave portion 22d and a second-click concave portion 22e are formed. A convex spring portion 24b of the click spring 24 is engaged with the two click concave portions 22d and 22e. The rotating member 22c is rotated and then the convex spring portion 24b of the click spring 24 is fit into one of the two click concave portions 22d and 22e. The position of the rotating member 22c is regulated at a predetermined position. That is, the click spring 24 and the two click concave portions 22d and 22e form a so-called click mechanism.

Incidentally, the forming positions of the two click recessed portions are set as follows. That is, when the clutch mechanism (not shown) is connected and the driving force of the driving motor (not shown) is set to be transmitted to the bending portion 12b (refer to Fig. 1) via the driving-force transmitting mechanism including the clutch mechanism, the position of the first-click concave portion 22d is set such that the click spring 24 is engaged with the first-click concave portion 22d of the rotating member 22c. Incidentally, the position of the rotating member 22c in this case is referred to as a connecting position in the following description.

On the other hand, when the transmission of the driving force of the driving motor (not shown) is released by the clutch mechanism (not shown), the position of the second-click concave portion 22e is set such that the click spring 24 is engaged with the second-click concave portion 22e of the rotating member 22c. Incidentally, the position of the rotating member 22c in this case is referred to as a released position in the following description.

In other words, at the initial step (connecting position; refer to Fig. 2) and the later step (released position; refer to Fig. 4) of the switching operating member 23x (specifically, rotating member 22e) which is operated by the operator, force-change sensing means is arranged to add, to the operator, the inconstant amount of change in force, that is, the sense of clicking. The click mechanism including the two click concave portions 22d and 22e of the rotating member 22c and the click spring 24 has functions of operation self-sensing means and perceiving means, serving as means for sensing the operation of the switching operating member 23x.

Referring to Fig. 3, incidentally, the knob member 23 of the switching operating member 23x is projected to the outside from the exterior surface of the control section 11. As a consequence, upon operating the switching operating member 23x so as to interrupt the clutch mechanism, the operator easily pinches the knob member 23. On the other hand, the externally-projected switching operating member 23x is position-regulated at the predetermined position by the above-mentioned click mechanism and therefore the operator's unintentional movement of the knob member 23 is prevented. The click mechanism thus functions as error-operation preventing means (error-operation preventing device) that prevents the erroneous operation of the switching operating member 23x.

With the endoscope 10 having the above-mentioned configuration, a description is given of the operation executed by operating the switching operating member 23x as follows.

In the state shown in Fig. 2, the clutch mechanism is in the connecting state, that is, the switching operating member 23x (the rotating member 22c) is at the connecting position. In the state; the click spring 24 is engaged with the first-click concave portion 22d. The operator pinches the knob portion 23 of the switching operating member 23x, and rotates the knob portion 23 in the direction along the arrow R shown in Fig. 2, e.g., in the clockwise direction in Fig. 2. Then, the rotating member 22c starts rotating in the same direction.

In this case, as mentioned above, the convex spring portion 24b of the click spring 24 is engaged with the first-click concave portion 22d of the rotating member 22c, and the repulsing force of the click spring 24 operates toward the outer circumference of the rotating member 22c in the direction shown by the arrow X1 in Fig. 2, that is, in the direction different from the rotating direction of the rotating member 22c (operating direction of the switching operating member 23x).

The rotating member 22c starts rotating in the clockwise direction and then the rotating member 22c keeps the contact state between the projected surface of the convex spring portion 24b of the click spring 24 and the recessed portion of the first-click concave portion 22d and simultaneously rotates against the repulsing force of the click spring 24. That is, being pressed-up in the direction shown by an arrow X2 in Fig. 2, the click spring 24 rotates.

In other words, being slid along the recessed portion of the first-click concave portion 22d, the projected portion of the convex spring portion 24b of the click spring 24 is pressed in the direction shown by the arrow X2 in Fig. 2. The convex spring portion 24b of the click spring 24 is out of the click concave portion 22d.

Therefore, the amount of slightly strong rotating force is required for a time from the start timing of the rotation of the switching operating member 23x to the timing at which the convex spring portion 24b is out of the click concave portion 22d. When the convex spring portion 24b is out of the click concave portion 22d, instantaneously, the amount of force for rotating the rotating member 22c changes and the rotating force is slightly weak. The change in amount of force means the change in strength of force which is sensed via the operator's finger pinching the switching operating member 23x.

Further, the operator rotates the switching operating member 23x in the same direction and then the rotating member 22c is rotated in the same direction. In this case, the convex spring portion 24b of the click spring 24 keeps the contact state with the outer circumference of the rotating member 22c by using the repulsing force in itself in the direction shown by the arrow X1 in Fig. 2 (direction from the outer circumference of the rotating member 22c to the center). In the state, the rotating member 22c continues the rotation thereof and then the second-click concave portion 22e is engaged with the click spring 24. At this time point, the operator stops the operation of the switching operating member 23x.

In this case, first, the projected surface of the convex spring portion 24b of the click spring 24 is slid along the caved surface of the second-click concave portion 22e from the outer circumference of the rotating member 22c. When the convex spring portion 24b of the click spring 24 is engaged with the second-click concave portion 22e, instantaneously, the amount of force for rotating the rotating member 22c changes. The change in amount of force means the change in strength of force which is sensed via the operator's finger pinching the switching operating member 23x.

When the convex spring portion 24b of the click spring 24 is engaged with second-click concave portion 22e, the repulsing force of the click spring 24 operates in the direction shown by the arrow X1, that is, in the direction different from a vector of the rotating direction of the rotating member 22c (operating direction of the switching operating member 23x). Therefore, in order to keep the state, the rotating member 22c is position-regulated in the rotating direction.

The switching operating member 23x is operated from the connecting position shown in Fig. 2 (in the state in which the convex spring portion 24b of the click spring 24 is engaged with the first-click concave portion 22d) and thus the rotating member 22c is rotated in the clockwise direction. Then, the shaft 22b is rotated in the same direction. The rotation of the shaft 22b operates a predetermined cam mechanism among the components partly constituting the clutch mechanism. A predetermined position on the driving-force transmitting line is moved in a predetermined direction.

Incidentally, the configuration of clutch mechanism for interrupting the driving force is not limited to the above-mentioned means using the cam mechanism. For example, the clutch cam mechanism portion can comprise a cam member cut by a tap into female screw, a shaft member cut by a die into male screw, and a rotation locking member for locking the rotation of the cam member. In this case, while the rotation locking member locks the rotation of the cam member, the shaft member is rotated. Then, the cam member moves in the thrust direction of the shaft member and the interruptive operation of clutch may be possible. With the above-mentioned configuration, the assembling and disassembling of the clutch cam mechanism portion may be improved and the rigidity of the clutch cam mechanism portion may be improved.

Referring back to Fig. 2, upon engaging the convex spring portion 24b of the click spring 24 with the second-click concave portion 22e (upon setting the rotating member 22c to the released position; refer to Fig. 4), the driving-force transmitting mechanism is partly disconnected. In other words, the clutch mechanism releases the transmission state of the driving force between the control section 11 and the bending portion 12b (refer to Fig. 1).

Therefore, the operator may sense the inconstant amount of change in force (the sense of clicking) upon rotating the rotating member 22c, and know whether or not the clutch mechanism operates. That is, the operator may sense the inconstant amount of change in force (the sense of clicking) occurred during the rotation of the switching operating member 23x, and know that the clutch mechanism operates.

In order to displace the clutch mechanism from the released state to the connecting state, the switching operating member 23x (the rotating member 22c) is rotated from the released position shown in Fig. 4 in the counterclockwise direction and is thus moved to the connecting position shown in Fig. 2. The operation in this case is a reverse of the operation as mentioned above, that is, the operation upon displaying the clutch mechanism from the connecting state to the released state, and the operation changes from the released state to the connecting state.

As mentioned above, according to the first embodiment, the click mechanism is operated interlocking with the switching operating member 23x, serving as the operating member for operating the clutch mechanism. It is set that the click mechanism (operation self-sensing means and perceiving means) generates the inconstant amount of change in force (the sense of clicking) at the start timing and the end timing of the operation of rotation of the switching operating member 23x, and the interruptive switching operation of the clutch mechanism is performed at the timing of the change in amount of force (the sense of clicking). Therefore, the operator may sense the inconstant amount of change in force (the sense of clicking) upon rotating the switching operating member 23x, thereby accurately knowing the operation of the clutch mechanism.

The knob member 23 of the switching operating member 23x is arranged to be externally projected from the exterior surface of the control section 11 (refer to Fig. 3). Hence, the operator easily pinches the knob member 23 of the switching operating member 23x for interrupting the clutch mechanism and the operability may be improved. On the other hand, the switching operating member 23x is position-regulated at one of predetermined positions (two positions for operating the clutch mechanism) by the click mechanism, serving as error-operation preventing means (error-operation preventing device). The unintentional operation of the switching operating member 23x is prevented despite the operator's intentions. Therefore, the higher safety is ensured.

Since the switching operating member is operated in the rotating direction, advantageously, the erroneous operation due to the undesired external force may be prevented, as compared with the case of operating a slide operating member in the straight direction. In addition, in the operating member operated in the rotating direction, the center of rotation is not moved and therefore the operating member is not moved in a wide range in the operation. Advantageously, the arrangement space is not relatively wide.

Next, a description is given of an electrical bending endoscope according to a second embodiment of the present invention.

Fig. 5 is a schematic diagram enlarging and showing a part of a control section of an electrical bending endoscope according to the second embodiment of the present invention, specifically showing the internal configuration in and around a switching operating member of the driving-force interrupting switching mechanism (clutch mechanism).

Referring to Fig. 5, the basic configuration according to the second embodiment is similar to that of according to the first embodiment. A specific description of the same configuration is omitted and only different portions will be described later.

The endoscope according to the second embodiment further comprises a detecting switch 26, serving as a detecting device that detects the operation of the clutch mechanism, in addition to the configuration of the endoscope according to the first embodiment. Referring to Fig. 5, the detecting switch 26 is integrated to the knob member 23 of a switching operating member 23Ax. In order to transmit a detecting signal outputted from the detecting switch 26, a lead 39, serving as a signal cable (signal line) extended from the detecting switch 26 is connected via the universal cable 18 to a motor control device 16A (refer to Fig. 5) among the units of the endoscope apparatus 1 (refer to Fig. 1) adopting an electrical bending endoscope 10A.

In this case, the switching operating member 23Ax is freely rotatably arranged. As mentioned above, upon arranging the detecting switch 26 to the switching operating member 23Ax, the lead 39 extended from it is arranged near the rotating member 22c or the like. Therefore, it is considered that the lead 39 is wound to a rotating member (22c or the like). Then, in consideration of this, according to the second embodiment, as shown in Fig. 5, a wire fixing member 40, serving as a signal-line fixing member; for partly fixing the lead 39 to a predetermined position is arranged in and around the switching operating member 23Ax (the rotating member 22c).

The wire fixing member 40 is arranged at a predetermined position near the supporting portion 22a formed integrally with an inner fixing member (not shown) of a control section 11A, along the outer circumference. The wire fixing member 40 is supported at both ends thereof by shaft members. In the center of the wire fixing member 40, an inner wiring member, a supporting portion 40a is arranged with a predetermined shape (e.g., column shape) so that the lead 39 is partly fixed to regulate the movable range.

Therefore, the lead 39 is partly fixed to a supporting portion 40a of the wire fixing member 40 so as to regulate the movable range of the lead 39. Thus, it is prevented that the inner wiring member, such as the lead 39, is wound to the movable member, such as the rotating member 22c.

As mentioned above, the lead 39 extended from the detecting switch 26 is connected to the motor control device 16A via the universal cable 18. Therefore, a predetermined detecting signal (signal indicating that the operation of the clutch mechanism is detected) generated by the detecting switch 26 is transmitted to the motor control device 16A.

In accordance therewith, a notifying member 27, serving as notifying means, is arranged on the side of the motor control device 16A so that the signal from the detecting switch 26 is received and predetermined warning or notification is displayed in a predetermined format.

The notifying member 27 can use various members, such as light-emitting member including a lamp or light-emitting diode (LED) for displaying the warning or notification with light, a sound generating member including a buzzer for warning and notification with sound, or a vibrating member for warning or notification caused by vibration. Other configurations are the same as those according to the first embodiment. Incidentally, the vibrations are generated by assembling a specific vibration generating device to the control section 11. In place of this, the motor control device 16 may rotate the motor forward and backward at the interval of a short period, thereby generating the vibration.

Hereinbelow, a description is given of the operation executed by operating the switching operating member 23Ax in an endoscope 10A according to the second embodiment.

First, similarly to the case according to the first embodiment, the operator pinches the knob member 23 of the switching operating member 23Ax which is to be rotated in the direction shown by an arrow R in Fig. 5, e.g., in the clockwise direction in Fig. 5. Then, the rotating member 22c starts the rotation in the same direction. Hereinbelow, via the same operation as that according to the first embodiment, the convex spring portion 24b of the click spring 24 is engaged with the second-click concave portion 22e. At the time (time for setting the rotating member 22c to the released position), the driving-force transmitting mechanism (not shown) is partly disconnected, and the clutch mechanism releases the transmission state of the driving force between the control section 11 and the bending portion 12b (refer to Fig. 1). At the same time, the detecting switch 26 generates a predetermined signal indicating that the clutch mechanism operates and the clutch is released. The signal is transmitted to the motor control device 16A via the lead 39.

The motor control device 16A receives the clutch release signal, thereby operating the notifying member 27. In this case, when the notifying member 27 is a light-emitting member, the notifying member 27 executes the light-on operation or light-off operation of a lamp or LED. When the notifying member 27 is a sound generating member, the notifying member 27 generates buzzer sound for a predetermined time. When the notifying member 27 is a vibrating member, the notifying member 27 generates vibrations for a predetermined time. Based on predetermined operation of the notifying member 27, the operator checks the release of the clutch mechanism.

When the clutch mechanism is displaced from the released state to the connecting state, similarly to the case according to the first embodiment, the switching operating member 23Ax (the rotating member 22c) is rotated from the released position in the counterclockwise direction, thereby being moved to the connecting position shown in Fig. 5.

In this case, when the switching operating member 23Ax (the rotating member 22c) is moved to a predetermined connecting position and the convex spring portion 24b of the click spring 24 is engaged with the first-click concave portion 22d, the detecting switch 26 generates a signal (clutch connecting signal) indicating that the clutch mechanism operates and then the clutch enters the connecting state. In response to this, the notifying member 27 of the motor control device 16A executes the light-off operation of a light-emitting member during the light-on operation, or executes the vibration of the vibrating member or the sound generation of the sound generating member. Thus, the operator checks that the clutch mechanism is connected.

Incidentally, the timing for generating a predetermined signal by the detecting switch 26 is not limited to the foregoing and may be different from this. That is, the timing for generating the clutch release signal or clutch connecting signal may be the timing just after starting the rotation by the switching operating member 23Ax.

As mentioned above, according to the second embodiment, the same advantages according to the first embodiment are obtained. In addition, the notifying member 27 is arranged to check, by the sense of hearing or sense of vision, that the state of the clutch mechanism is switched, and the detecting switch 26 is arranged to detect the operation of the clutch mechanism interlocking with the rotation of the switching operating member 23Ax. Thus, the notifying member 27 is operated based on the detecting signal from the detecting switch 26 and the operator checks the state of the clutch mechanism with more improved accuracy.

If the endoscope 10A is erroneously fallen during the use thereof and then the clutch mechanism is operated at an unintentional timing, the operator checks such a fact by at least one of the sense of hearing and the sense of vision. The operability and the safety are more improved.

Further, the wire fixing member 40 is arranged to partly fix the lead 39 and regulate the movable range, thereby preventing the winding of the inner wiring member, such as the lead 39 to the movable member, such as the rotating member 22c. Thus, the electrical bending endoscope ensures higher safety.

According to the second embodiment, the notifying member 27, serving as notifying means, is arranged to the side of the motor control device. However, the present invention is not limited to this. For example, in the endoscope 10A, a scope for observation of forward viewing (not shown) generally-arranged displays operation warning within a field-of-view for observation, or a display device displays predetermined data on a display screen thereof, and various formats are considered and the individual formats may be easily combined.

Next, a description is given of an electrical bending endoscope according to a third embodiment of the present invention.

Fig. 6 is a schematic diagram enlarging and showing a part of a control section of an electrical bending endoscope according to the third embodiment of the present invention, specifically showing the internal configuration in and around a switching operating member of the driving-force interrupting switching mechanism (clutch mechanism). Fig. 7 is a sectional view along a line 7-7 shown in Fig. 6, specifically showing the details of the switching operating member.

Referring to Fig. 6, the configuration according to the third embodiment is similar to that of according to the first embodiment. The same configuration is not described and only different portions will be described later.

According to the first embodiment, as mentioned above, only after one-step operation, that is, the switching operating member 23x is rotated in the direction shown by the arrow R in Fig. 2, and the click mechanism, serving as error-operation preventing means (error-operation preventing device) and operation self-sensing means and perceiving means, releases the position regulation. On the other hand, according to the third embodiment, a position regulating mechanism, serving as error-operation preventing means (error-operation preventing device), regulates the position of a switching operating member 23Bx and prevents the erroneous operation, and releases the position regulation of the switching operating member 23Bx, after two-step operation.

That is, according to the third embodiment, unlike the first embodiment, the click mechanism according to the first embodiment is not used and, in place of this, a position regulating mechanism 30 is arranged.

The position regulating mechanism 30 comprises: a locking lever 28, serving as a regulating member of the switching-operation position which is a locking member integrated to the knob member 23 and rotatable within a predetermined range; and a locked portion 29 which is formed throughout the movable range of the locking lever 28 and has two locked steps 29d and 29e for engaging the locking lever 28.

Referring to Fig. 7, the locking lever 28 of the position regulating mechanism 30 comprises: a lever member 28a having the cross section which is L-shaped; and an extension spring 28d that makes repulse the lever member 28a.

The locked portion 29 is integrally formed at a predetermined position of the inner fixing member of a control section 11B in an endoscope 10B, that is, at a predetermined position along the outer circumference of a supporting portion 22Ba in the control section 11B.

The locked portion 29 is formed throughout the movable range of the locking lever 28. Near one end of the locked portion 29, a first step 29d (connecting position) is formed. Near the other end of the locked portion 29, a second step 29e (released position) is formed. The two steps 29d and 29e are formed such that a predetermined position of the lever member 28a of the locking lever 28 is engaged with the two steps 29d and 29e. When the lever member 28a is rotated together with the switching operating member 23Bx and is arranged at a predetermined position, the predetermined position is engaged with one of the two steps 29d and 29e. The engagement between them regulates the position of the switching operating member 23Bx.

Thus, the lever member 28a is coaxially supported by a shaft 28e to be freely rotatable to the fixing position of the knob member 23 in the direction shown by an arrow Rx in Fig. 7 within a predetermined range. In this case, a short-arm portion 28b of the lever member 28a is arranged so as to be in parallel with the knob member 23. A long-arm portion 28c is arranged such that at least the end thereof is in the direction orthogonal to the knob member 23 and is parallel with step surfaces of the steps 29d and 29e of the locked portion 29.

One end of the extension spring 28d is fixed at a predetermined position on the side of short-arm portion 28b of the lever member 28a. The other end of the extension spring 28d is fixed to a predetermined position of the knob member 23. As a consequence, the extension spring 28d is extended with repulsing force in the extending direction of space between the short-arm portion 28b and the knob member 23.

Therefore, the lever member 28a is always repulsed in the counterclockwise direction along the arrow Rx shown in Fig. 7 with the shaft 28e as center, that is, the long-arm portion 28c is always repulsed to the surface of the locked portion 29. In other words, the long-arm portion 28c is always repulsed in the direction shown by an arrow X4 in Fig. 7. Hence, the long-arm portion 28c of the lever member 28a in the locking lever 28 always comes into contact with the surface of the locked portion 29 in the normal state.

The above-structured locking lever 28 is integrated to a predetermined position of the switching operating member 23Bx. In this case, the lever member 28a is arranged such that the distal end of the long-arm portion 28c is extended to the outer circumference thereof, beyond the supporting portion 22Ba. Therefore, a circumferential groove 22Baa (refer to Fig. 6) is pierced through the supporting portion 22Ba throughout the rotating range of the lever member 28a.

The switching operating member 23Bx according to the third embodiment is covered with a knob cover 31, serving as a protecting member. The knob cover 31 is formed by a waterproof member constituted of rubber or the like. The knob cover 31 is watertightly and rotatably arranged to an exterior member 11Ba of the control section 11B. Other configurations are the same as those according to the first embodiment.

With the endoscope 10B having the above-mentioned configuration according to the third embodiment, the operation executed by operating the switching operating member 23Bx is briefly described hereinbelow.

First, similarly to the case according to the first embodiment, the operator pinches the knob member 23 of the switching operating member 23Bx from the outer surface of the knob cover 31 in the state shown in Fig. 6 (in this case, the state for connecting the clutch mechanism) (first operation), and simultaneously rotates the knob member 23 in the direction shown by the arrow R in Fig. 6, e.g., in the clockwise direction in Fig. 6 (second operation).

Upon pinching the knob member 23 of the switching operating member 23Bx, the short-arm portion 28b of the lever member 28a in the locking lever 28 is pinched together with the knob member 23. As a consequence, the lever member 28a is rotated against the repulsing force of the extension spring 28d in the direction shown by the arrow Rx shown in Fig. 7 with the shaft 28e as center, that is, in the clockwise direction. Therefore, the short-arm portion 28b of the lever member 28a is moved to the side of the knob member 23 and the engagement of the long-arm portion 28c with the first step 29d is released (first operation).

In the state, that is, the knob member 23 and the lever member 28a are pinched from the outside of the knob cover 31 and are simultaneously rotated in the clockwise direction (second operation).

The switching operating member 23Bx is rotated to some degree and then the end of the long-arm portion 28c in the lever member 28a is completely separated from the first step 29d. Therefore, in the state, even if the amount of force for pinching the knob member 23 is released, this does not influence on the rotation of the switching operating member 23Bx.

When the switching operating member 23Bx starts rotating, the clutch mechanism starts the release operation of the connecting state. When the lever member 28a is engaged with the second step 29e (is set to the released position), the driving-force transmitting mechanism (not shown) is partly disconnected and the clutch mechanism releases the transmission of driving force between the control section 11B and the bending portion 12b (refer to Fig. 1).

Therefore, the operator senses the change in amount of force upon engaging the lever member 28a with the second step 29e and knows whether or not the clutch mechanism is operated. That is, the operator senses the change in amount of force for rotating the switching operating member 23Bx, thereby knowing the operation of the clutch mechanism.

On the other hand, when clutch mechanism is displaced from the released state to the connecting state, similarly to the case according to the first embodiment, the switching operating member 23Bx (rotating member 22c) is at the released position and the knob member 23 and the lever member 28a are simultaneously pinched. Then, the knob member 23 and the lever member 28a are rotated in the counterclockwise direction and are moved to the connecting position shown in Fig. 6.

In this case, when the switching operating member 23Bx (rotating member 22c) is moved to a predetermined connecting position and the lever member 28a is engaged with the first step 29d, the clutch mechanism operates, thereby connecting the clutch. The operator senses the change in amount of force in this case, thereby confirming the connection of the clutch mechanism.

As mentioned above, according to the third embodiment, the same advantages according to the first embodiment are obtained.

Further, the switching operating member 23Bx according to the third embodiment, at the connecting position for keeping the connecting state of the clutch mechanism and the released position for releasing the connecting state of the clutch mechanism, the locking lever 28 is integrally arranged to regulate the rotation to be done without fail and prevent the erroneous operation. Further, in order to rotate the switching operating member 23Bx, the operation for releasing the locking of the locking lever 28, that is, the operation (first operation) for pinching the knob member 23 and the lever member 28a is executed. Thereafter, the rotation of the switching operating member 23Bx (second operation) is executed.

In other words, according to the third embodiment, in the operation for switching the state of the clutch mechanism, input means needs two or more steps of continuous operation (serial operation). Therefore, even if unintentional external force is applied to the switching operating member 23Bx, undesired rotation may be prevented. This contributes to the prevention of the erroneous and unnecessary switching of the state of the clutch mechanism, and functions as error-operation preventing means (error-operation preventing device).

The knob portion 23 of the switching operating member 23Bx according to the third embodiment is pinched together with the locking lever 28, and the engagement between the lever member 28a of the locking lever 28 and the first step 29d or second step 29e of the locked portion 29 is released. However, the means for releasing the engagement is not limited to this, and the knob portion 23 and the lever member 28a are movable together therewith in the switching operating member 23Bx, and the switching operating member 23Bx is moved in the axial direction before the rotation of the switching operating member 23Bx, thereby releasing the engagement of the lever member 28a. In this case, the knob cover 31 is extendable in the axial direction and is watertightly arranged to the exterior member 11Ba.

Next, a description is given of an electrical bending endoscope according to a fourth embodiment of the present invention.

Fig. 8 is a schematic diagram enlarging and showing a part of a control section of the electrical bending endoscope driving-force interrupting switching mechanism (clutch mechanism), specifically showing the internal configuration in and around a switching operating member according to the fourth embodiment of the present invention.

Referring to Fig. 8, the basic configuration according to the fourth embodiment is similar to that of according to the second embodiment. The same configuration is not described and only different portions will be described later.

According to the fourth embodiment, differently, a holding detecting switch 32, serving as a detecting device, is arranged, in place of the detecting switch 26 according to the second embodiment. The detecting switch 26 according to the second embodiment detects the operating state of the clutch mechanism. However, the holding detecting switch 32 according to the fourth embodiment, detects whether or not the operator holds a switching operating member 23Cx.

Similarly to the detecting switch 26 according to the second embodiment, the holding detecting switch 32 is integrally arranged to the knob member 23 of the switching operating member 23Cx.

Similarly, the lead 39, serving as a signal cable, extended from the holding detecting switch 32 is connected via the universal cable 18 to the motor control device 16A (refer to Fig. 8) as a unit of the endoscope apparatus 1 (refer to Fig. 1) using an electrical bending endoscope 10C.

Similarly to the second embodiment, the lead 39 is partly fixed to the supporting portion 40a of the wire fixing member 40, thereby regulating the movable range. Thus, it is prevented that the inner wiring member, such as the lead 39 is wound to the movable member of the rotating member 22c. Other configurations are the same as those according to the second embodiment.

With the endoscope 10C having the above-mentioned configuration according to the fourth embodiment, the operation for operating the switching operating member 23Cx is as follows.

First, the operator executes predetermined operation and then the holding detecting switch 32 is operated, thereby generating a predetermined holding detecting signal indicating that the switching operating member 23Cx is held by the operator. The setting detecting signal is transmitted to the motor control device 16A via the lead 39.

Similarly to the first and second embodiments, the operator pinches the knob member 23 of the switching operating member 23Cx and rotates the knob member 23 in the direction shown by an arrow R in Fig. 8, e.g., in the clockwise direction in Fig. 8. Then, the rotating member 22c starts to be rotated in the same direction. Hereinbelow, after undergoing the same operations as those according to the first and second embodiments, the convex spring portion 24b of the click spring 24 is engaged with the second-click concave portion 22e. At the time (time for setting the rotating member 22c to the released position), similarly to the first embodiment, the driving-force transmitting mechanism (not shown) is partly disconnected and the clutch mechanism releases the transmission of driving force between a control section 11C and the bending portion 12b (refer to Fig. 1). Therefore, the operator checks the release of the clutch mechanism by the sense of clicking in this case.

The operator operates the holding detecting switch 32, thereby generating the predetermined setting detecting signal as mentioned above. Further, the setting detecting signal is transmitted to the motor control device 16A via the lead 39. The motor control device 16A receives the setting detecting signal and thereafter checks whether or not the switching operating member 23Cx is rotated for a predetermined time or whether or not the clutch mechanism is released. Incidentally, for example, detecting means that detects the state of the switching operating member 23Cx or detecting means that detects the state of the clutch mechanism is arranged, and the detecting signal is transmitted to the motor control device 16A via a predetermined lead.

When it is checked that the switching operating member 23Cx is not rotated, the motor control device 16A determines that an abnormal operation is performed and then activates the notifying member 27. The notifying member 27 is similar to that according to the second embodiment and has the same operations as those according to the second embodiment.

On the other hand, when the clutch mechanism is displaced from the released state to the connecting state, similarly to the case according to the first embodiment, the switching operating member 23Cx (rotating member 22c) is rotated from the released position in the counterclockwise direction and is moved to the connecting position shown in Fig. 8.

In this case, the switching operating member 23Cx (the rotating member 22c) is moved to a predetermined connecting position and then the operator confirms the connection of the clutch mechanism by the sense of clicking generated at the timing for engaging the convex spring portion 24b of the click spring 24 with the first-click concave portion 22d. The operator performs a predetermined operation so as to stop the operation of the holding detecting switch 32.

When the operation for stopping the operation of the holding detecting switch 32 is not executed for a predetermined time after setting the switching operating member 23Cx to the connecting position, the motor control device 16A activates the notifying member 27. With the notice, the operator recognizes that the switching operation of the clutch mechanism does not end.

As mentioned above, according to the fourth embodiment, the same advantages as those according to the second embodiment are obtained. Further, according to the fourth embodiment, even if applying unintentional external force to the switching operating member 23Cx, the operation of the click mechanism prevents undesired operation of the switching operating member 23Cx.

In performing the interrupting operation of the clutch mechanism, input means needs at least two operations in parallel, that is, the operation of the holding detecting switch 32 and the rotation of the switching operating member 23Cx. Thus, the interrupt by the clutch mechanism is not executed without the operator's intention and the safety is ensured.

Incidentally, the clutch mechanism according to the first to the fourth embodiments is a mechanical clutch mechanism that interrupts the operation of the clutch with the mechanical structure. According to the fourth embodiment, the clutch mechanism is easily structured by using an electromagnetic clutch that is electrically operated.

That is, in the endoscope using the electromagnetic clutch, only upon receiving operating signals from both the holding detecting switch 32 and an operation detecting switch that detects the electromagnetic clutch, the electromagnetic clutch may be operated. With the above-mentioned configuration, the same advantages are obtained.

Next, a description is given of an electrical bending endoscope according to a fifth embodiment of the present invention.

Fig. 9 is a schematic diagram enlarging and showing a part of a control section of the electrical bending endoscope driving-force interrupting switching mechanism (clutch mechanism), specifically mainly showing a switching operating member according to the fifth embodiment of the present invention.

According to the fifth embodiment, the electrical bending endoscope is similar to that of according to the first embodiment. The same configuration is not shown, a specific description is not given, and only different portions will be described later.

Referring to Fig. 9, an electrical bending endoscope 10D according to the fifth embodiment, in place of the switching operating members (23x, 23Ax, 23Bx and 23Cx) according to the first to fourth embodiments, a switching operating member 23Dx, serving as additional input means, is arranged to be detachably coupled to a control section 11D.

In the interrupting operation of a clutch mechanism (not shown) in the endoscope 10D according to the fifth embodiment, a shaft 22Db interlocking with a main mechanism portion of the clutch mechanism is directly rotated.

That is, according to the fifth embodiment, an L-shaped key-wrench-type switching operating member 23Dx is arranged, as wrench for general hexagon bolt. The switching operating member 23Dx uses a connecting member, such as a chain, serving as a connecting device, at a predetermined position of the control section 11D and is connected to be integrated to the control section 11D. Thus, the loss of the switching operating member 23Dx is prevented.

In accordance therewith, at the distal end of the shaft 22Db, a hexagon engaging hole 22Dba is formed so as to match with the wrench shape (hexagon shape) of the switching operating member 23Dx.

The shaft 22Db is arranged to be externally projected from an opening 11Dd pierced at a predetermined position of the side portion of the control section 11D of the endoscope 10D. The opening 11Dd is watertightly covered by a cover member 33 which is constituted of a rubber member or the like and is detachably arranged to the control section 11D.

Similarly to the switching operating member 23Dx, the cover member 33 is integrally connected at a predetermined position of the control section 11D by using a connecting member, e.g., a chain in order to prevent the loss of the cover member 33. Incidentally, referring to Fig. 9, the detachment of the cover member 33 for covering the shaft 22Db is shown.

A predetermined index M is arranged at a predetermined position of the distal end of the shaft 22Db. Corresponding to the arrangement, two indexes N1 and N2 are arranged near the outer circumference of the shaft 22Db on the side of the control section 11D. In this case, referring to Fig. 9, the index N1 designated by "Δ" denotes the connecting position where the clutch mechanism is in the connecting state. Further, referring to Fig. 9, the index N2 designated by "O" denotes the released position where the clutch mechanism is in the released state.

The endoscope 10D with the above-mentioned configuration according to the fifth embodiment, the operation in the interrupting operation of the clutch mechanism is as follows.

First, the operator detaches the cover member 33 of the control section 11D. Subsequently, the operator engages the switching operating member 23Dx with an engaging hole 22Dba of a shaft 22Db. In this case, an index M of the shaft 22Db is at the position matching with an index N1, that is, at the connecting position (in the state shown in Fig. 9).

From the state, the switching operating member 23Dx is rotated in a predetermined direction (e.g., clockwise direction) along an arrow R shown in Fig. 9 within a predetermined range, that is, to the position (released position) where the index M of the shaft 22Db matches with the index N2. Thus, the clutch mechanism enters the released state.

The cover member 33 is attached to a predetermined position for covering the opening 11Dd of the control section 11D.

On the other hand, in order to displace the clutch mechanism from the released state to the connecting state, similarly, the cover member 33 is detached, and the switching operating member 23Dx is thereafter engaged with the connecting hole 22Dba of the shaft 22Db and is simultaneously rotated from the released position within a predetermined range in the counterclockwise direction along the arrow R shown in Fig. 9. That is, the switching operating member 23Dx is rotated to the position (released position) where the index M of the shaft 22Db matches with the index N1. Hence, the clutch mechanism enters the connecting state.

The cover member 33 is attached to a predetermined position for covering the opening 11Dd of the control section 11D.

As mentioned above, according to the fifth embodiment, the switching operating member 23Dx is freely detachable to the control section 11D, and the shaft 22Db for interrupting the clutch mechanism is covered with the cover member 33 in the normal state. Therefore, the clutch mechanism is operated only by the operator's intention, and the unintentional operation of the clutch mechanism may be prevented in an unnecessary case. In the necessary time, predetermined interrupting operation of the clutch mechanism needs to be executed, and the operator may always recognize the state of the clutch mechanism.

Next, a description is given of an electrical bending endoscope according to a sixth embodiment.

Fig. 10 is a schematic diagram enlarging and showing a part of a control section of the electrical bending endoscope driving-force interrupting switching mechanism (clutch mechanism), specifically showing the configuration in and around a switching operating member according to the sixth embodiment of the present invention. Fig. 11 is a side view of the main portion, being cut out and seen in the direction of an arrow C shown in Fig. 10, in and around the switching operating member, serving as a part of the control section shown in Fig. 10.

The basic configuration according to the sixth embodiment is similar to that of according to the first embodiment. According to the sixth embodiment, referring to Fig. 10, a knob member 23E partly forming a switching operating member 23Ex is different from that according to the first embodiment. Therefore, the same configuration as that according to the first embodiment is not described in detail and only different portions will be described.

Referring to Fig. 10, the knob member 23E of the switching operating member 23Ex according to the sixth embodiment is circular-shaped so as to be arranged in the supporting portion 22a that is integrally formed to a fixing member of a control section 11E. In the center of the knob member 23E, a knob portion 23Ea is formed. Thus, on the inner circumference of the knob member 23E, half-moon-shaped holes 23Eb are pierced through both ends of the knob portion 23Ea. The holes 23Eb are arranged so that the operator easily pinches the knob portion 23Ea in the operation of the switching operating member 23Ex.

Therefore, referring to Fig. 11, the knob member 23E of the switching operating member 23Ex according to the sixth embodiment is arranged without being externally projected from the exterior surface of the control section 11E, in other words, the knob member 23E is arranged at a predetermined position of the control section 11E in the embedded form (as embedded input means). Other configurations and operations are the same as those according to the first embodiment.

As mentioned above, according to the sixth embodiment, the same advantages according to the first embodiment are obtained. The knob member 23E of the switching operating member 23Ex is embedded in the control section 11E. Therefore, even if unintentional external force is applied in and around the switching operating member 23Ex, the unintentional rotation of the switching operating member 23Ex is prevented. That is, according to the sixth embodiment, as long as the operator does want to rotate the switching operating member 23Ex, the clutch mechanism is not operated. Therefore, the safety may be ensured without deterioration of preferable operability.

The knob member 23E according to the sixth embodiment is embedded in the control section 11E, thereby functioning as error-operation preventing means (error-operation preventing device) and as switching operating member protecting means for protecting the knob member 23E.

Next, a description is given of an electrical bending endoscope according to a seventh embodiment of the present invention.

Figs. 12 and 13 are diagrams cutting-out and showing in and around a switching operating member, serving as a part of a control section in an electrical bending endoscope according to the seventh embodiment of the present invention. Fig. 12 is a side view thereof. Fig. 13 is a schematically perspective view seeing from the left of the switching operating member.

The basic configuration according to the seventh embodiment is similar to that of according to the first embodiment. According to the seventh embodiment, referring to Figs. 12 and 13, unlike the first embodiment, a protective barrier 34 is added, as switching operating member protecting means (switching-operation protecting means), which partly covers and protects the outer circumference of the knob member 23 partly forming the switching operating member. Therefore, the same configuration as that according to the first embodiment is not described in detail and only different portions will be described.

As mentioned above, according to the seventh embodiment, the protective barrier 34 which partly covers the outer circumference of the knob member 23 of the switching operating member is formed such that the top surface of a supporting portion 22a of a control section 11F in an endoscope 10F is extended.

The protective barrier 34 is constituted of the substantially same material as that of the control section 11F, and is formed by arranging another forming member on the top of the supporting portion 22a with means, such as an adhesive, or is integrally formed to the supporting portion 22a.

Incidentally, the protective barrier 34 is arranged so as protect at least the operating direction (rotating direction) of the knob member 23 of the switching operating member. Other configurations and operations are the same as those according to the first embodiment.

As mentioned above, according to the seventh embodiment, the same advantages according to the first embodiment are obtained. The protective barrier 34 is arranged to a part of the outer circumference of the knob member 23 of the switching operating member. Therefore, if unintentional external force is applied to the operating (rotating) direction of the knob member 23, the rotation of the knob member 23 may be prevented. Therefore, the operation of the clutch mechanism in the unintentional case is prevented, and higher safety may be ensured without deterioration in preferable operability.

Next, a description is given of an electrical bending endoscope according to an eighth embodiment of the present invention.

Fig. 14 is a sectional view enlarging and showing a part of a control section in an electrical bending endoscope, specifically schematically showing the internal configuration in and around switching operating member of a driving force interrupting switching mechanism (clutch mechanism) according to the eighth embodiment of the present invention.

The basic configuration according to the eighth embodiment is similar to that of according to the first embodiment. According to the eighth embodiment, referring to Fig. 14, unlike the first embodiment, an elastic member 35 for connecting a knob member 23G partly forming the switching operating member and a shaft 22Gb interlocking with a main mechanism portion of the clutch mechanism is arranged therebetween so that the operating force from the side of the switching operating member can be transmitted to the side of the clutch mechanism. Therefore, the same configuration as that according to the first embodiment is not described in detail and only different portions will be described.

According to the eighth embodiment, the knob member 23G of the switching operating member is arranged in an opening 11Gb formed at a predetermined position of an exterior member 11Ga of a control section 11G. In this case, between the outer circumference of the knob member 23G and the inner circumference of the opening 11Gb, an O ring 36 with a predetermined shape is arranged. Due to the O ring 36, the knob member 23G is watertightly arranged to the opening 11Gb.

As mentioned above, between the knob member 23G and the shaft 22Gb, an elastic member 35 is arranged such that the operating force (rotating force) from the knob member 23G via the elastic member 35 can be transmitted to the side of the clutch mechanism.

In this case, the elastic member 35 comprises an elastic spring and the like, and is formed to absorb the amount of force in the axial direction and the amount of force from the diagonal direction to the axial direction. That is, for example, while the amount of rotating force in the clockwise direction is distributed, a predetermined amount of rotating force (e.g., the amount of rotating force in the rotation at a rotating angle of 10° to 20°) of the amount of rotating force in the counterclockwise direction is absorbed and the amount of rotating force is not transmitted to the knob member 23G.

Therefore, when the rotation in the direction for tightening the elastic member 35 of the knob member 23G has the amount over a predetermined amount (the amount of force at a rotating angle of 10° to 20°) and the elastic member 35 cannot absorb the amount of rotation, the elastic member 35 operates such that the amount of rotating force may be transmitted to the shaft 22Gb. Thus, the operating force (rotating force) from the side of the knob member 23G is transmitted to the side of the mechanism via the elastic member 35. Other configurations and operations are the same as those according to the first embodiment.

As mentioned above, according to the eighth embodiment, the same advantages according to the first embodiment are obtained. The elastic member 35 is arranged between the knob member 23G and the shaft 22Gb. Therefore, when applying the load due to the unintentional external force to the knob member 23G of the switching operating member, the vector direction of the load applied by the operation of the elastic member 35 changes. That is, in this case, the elastic member 35 functions as load distributing means (load distributing mechanism) for distributing the applied load.

Therefore, the load from the external force is not transmitted to the clutch mechanism via the shaft 22Gb. Therefore, it may be possible to prevent the influence, on the units forming the control section 11G, by the load applied to the switching operating member from the external force.

Next, a description is given of an electrical bending endoscope according to a ninth embodiment.

Fig. 15 is a schematic diagram enlarging and showing the configuration of a part of a control section in the electrical bending endoscope, specifically showing the internal configuration in and around switching operating member of a driving-force interrupting switching mechanism (clutch mechanism).

Referring to Fig. 15, the electrical bending endoscope 10 according to the ninth embodiment comprises means for precisely positioning the clutch mechanism unit 25, thereby obtaining the watertightness at the attaching position of the switching operating unit for interrupting the clutch mechanism.

The basic configuration according to the ninth embodiment is similar to that of according to the first embodiment. Therefore, the same configuration as that according to the first embodiment is not described in detail and only different portions will be described hereinbelow.

Referring to Fig. 15, in the endoscope 10 according to the ninth embodiment, the clutch mechanism unit 25 is arranged at a predetermined position in the control section 11. The clutch mechanism unit 25 has a switching operating member 23x for interrupting operation. As mentioned above according to the first embodiment, the switching operating member 23x is arranged to the shaft 22b interlocking with a main mechanism portion (not shown) of the clutch mechanism unit 25 in the control section 11.

The knob member 23 is externally exposed from an opening 11d pierced at a predetermined position of the side portion of the control section 11, and the switching operating member 23x is partly projected a little from the surface of the exterior member of the control section 11.

The watertightness is necessary to be ensured at the portion attaching the switching operating member 23x to the control section 11. In order to ensure the watertightness and maintain the state, to an opening 11d of the control section 11, the attaching position of the clutch mechanism unit 25 fixed to the inner fixing member of the control section 11 and the attaching position of the switching operating member 23x arranged to the clutch mechanism unit 25 are to be precisely arranged.

According to the ninth embodiment, the form of the material of the supporting portion for fixing and supporting the clutch mechanism unit 25 in the control section 11 is devised to solve the problem.

Referring to Fig. 15, according to the ninth embodiment, the clutch mechanism unit 25 is elastically supported in the control section 11 by two clutch mechanism supporting levers 37, serving as supporting mechanisms. The clutch mechanism supporting lever 37 comprises: a short-arm portion having one end thereof fixed to one side surface of the clutch mechanism unit 25; and a long-arm portion that is extended from the other end of the short-arm portion and is position-regulated by predetermined means near a grip portion 11e in the control section 11.

Near the distal end of the long-arm portion, a projected portion 37bb is arranged to be externally projected upon arranging the clutch mechanism supporting lever 37 in the control section 11.

Corresponding to the projected portion 37bb, an inner fixing member 11b near the grip portion 11e in the control section 11 has a recessed portion 11bb having an opening with the size for engagement of the projected portion 37bb.

The projected portion 37bb of the long-arm portion of the clutch mechanism supporting lever 37 is engaged with recessed portion 11bb of the inner fixing member 11b of the grip portion 11e in the control section 11.

According to the ninth embodiment, in the control section 11 with the above-mentioned configuration, the clutch mechanism unit 25 is fixed and supported by the clutch mechanism supporting lever 37, and the projected portion 37bb of the clutch mechanism supporting lever 37 is arranged. Meanwhile, the recessed portion 11bb engaged with the projected portion 37bb is arranged to the inner fixing member 11b of the control section 11. Thus, the clutch mechanism unit 25 may be precisely positioned. Therefore, the positioning between the clutch mechanism unit 25 and the attaching portion (opening 11d) of the switching operating member 23x may be precise.

When the load from the unintentional external force is applied to the control section 11, the load is transmitted to the clutch mechanism supporting lever 37 via an engaging portion of the recessed portion 11bb and the projected portion 37bb. Therefore, the load may not be easily transmitted to the switching operating member 23x.

In the electrical bending endoscope according to the embodiments, the components, such as the clutch mechanism and the driving-force transmitting mechanism including the clutch mechanism are arranged in the control section. The components increase a relatively large weight of the control section.

The operator operates the control section and, in many cases, he/she has the control section in one hand.

Therefore, the improvement may also be necessary to prevent a fact that the user is not conscious of the heavy feeling upon supporting and using the control section in one hand.

Generally, when people grip a thing having the weight in one hand, the heavy feeling is varied depending on the shape of thing. That is, upon gripping the thing having the gravity center which is concentrated near the center thereof, it is well-known that the heavy feeling is reduced by gripping the gravity center of thing in and around the center of the palm. That is, the load moment to the wrist is reduced by placing the gravity center of thing near the center of the palm.

Then, in the electrical bending endoscope according to the above embodiments, the arrangement of components including the clutch mechanism arranged in the control section is devised. Then, referring to Fig. 16, a center of gravity position CB is set near the center of the hand of the operator which grips and uses the control section (refer to reference numeral CB).

With the above-mentioned configuration, the heavy feeling to the operator's hand having the control section may be reduced. Thus, the operator may stably hold the control section and this may contribute to the improvement in operability.

Incidentally, the present invention is not limited to the above embodiments and can be variously modified without departing from the essentials of the present invention.

### Industrial Applicability

As mentioned above, according to the present invention, it may be possible to provide an electrical bending endoscope, in which the connection and disconnection of a transmitting line of the driving force due to the operation of the clutch mechanism used for the electrical bending endoscope may be certainly switched, the setting state may be kept accurately, the switching to the state set by the user may be known accurately in the switching operation, and the operability may be improved and the safety may be ensured.

According to the present invention, an electrical bending endoscope is provided, in which the amount of external force to the switching operating member does not cause the switching operation easily and the higher safety is ensured.

## Claims

1. An electrical bending endoscope comprising:
driving means, serving as a supply source of driving force;
driving-force transmitting means that transmits the driving-force supplied from the driving means to a bending portion to be bent;
driving-force interrupting means that is arranged to a transmitting line of the driving-force transmitted from the driving means to the bending portion and interrupts the transmission of the driving force;
switching operating means that is interlocked with the driving-force interrupting means, to interrupt the transmission of the driving force via the driving-force interrupting means upon its operation; and
error-operation preventing means that prevents unintentional operation of the switching operating means.

2. An electrical bending endoscope comprising:
an elongated insertion section having a bending portion that is bendable within a predetermined range;
a control section that is arranged to the proximal end of the insertion section;
a driving source that generates the driving force for bending the bending portion;
a driving-force transmitting mechanism that transmits the driving force supplied from the driving source to the bending portion;
a driving-force interrupting mechanism that is arranged to a transmitting line of the driving force transmitted from the driving source to the bending portion and interrupts the transmission of the driving force;
a switching operating member that is arranged to the control section and mechanically connected to the driving-force interrupting mechanism, to interrupt the transmission of the driving force via the driving-force interrupting mechanism upon its operation; and
an error-operation preventing device that prevents unintentional operation of the switching operating member.

3. The electrical bending endoscope according to Claim 2, wherein the switching operating member is movable, and
the error-operation preventing device comprises perceiving means that senses a predetermined position on a moving line of the switching operating member.

4. The electrical bending endoscope according to Claim 3, wherein the switching operating member is movable in the rotating direction.

5. The electrical bending endoscope according to Claim 3, wherein the perceiving means senses the position of the switching operating member by generating the inconstant amount of change in force.

6. The electrical bending endoscope according to Claim 3, further comprising:
a detecting device that detects the interrupt state of the driving-force interrupting mechanism and outputs a detecting signal and is integrally arranged to the switching operating member;
a signal line that is extended from the detecting device and transmits the detecting signal outputted from the detecting device; and
a signal-line fixing member that fixes at least one part of the signal line to regulate a moving range of the signal in accordance with the movement of the switching operating member.

7. The electrical bending endoscope according to Claim 3, wherein the perceiving means comprises a regulating member of the switching-operation position that regulates the movement of the switching operating member at the predetermined position and releases the regulation by the operation in the direction different from the moving direction of the switching operating member, the position of the switching operating member is sensed by operating the regulating member of switching-operation position in the direction different from the moving direction of the switching operating member.

8. The electrical bending endoscope according to Claim 3, wherein the perceiving means comprises:
a detecting device that detects whether or not the operator is in contact with the switching operating member; and
notifying means that notifies that the switching operating member is not operated within a predetermined period though the detecting device detects the contact state.

9. The electrical bending endoscope according to Claim 2, wherein the switching operating member is capable of being mechanically disconnected from the driving-force interrupting mechanism.

10. The electrical bending endoscope according to Claim 9, further comprising:
a connecting device that prevents the switching operating member from removing from the control section, when the switching operating member is mechanically disconnected from the driving-force interrupting mechanism.

11. The electrical bending endoscope according to Claim 2, wherein the switching operating member is arranged so as not to project from the exterior surface of the control section.

12. The electrical bending endoscope according to Claim 2, further comprising,
a protective barrier that protects the switching operating member so as to prevent the influence from the unintentional operation from the direction along the exterior surface of the control section.

13. The electrical bending endoscope according to Claim 3, further comprising,
a load distributing mechanism that prevents the transmission, to the driving-force interrupting mechanism, of the amount of force applied to the switching operating member, when the amount of force for moving the switching operating member does not reach a predetermined amount or more.

14. The electrical bending endoscope according to Claim 2, further comprising,
a supporting mechanism that supports the driving-force interrupting mechanism so as to be able to change the position in the control section.

15. The electrical bending endoscope according to Claim 2, further comprising,
a protecting member provided so as to watertightly protect the switching operating member, without disturbing the operation of the switching operating member.

16. The electrical bending endoscope according to Claim 2, wherein the driving-force interrupting mechanism is arranged such that the center of gravity position of the control section including the driving-force interrupting mechanism is on the normal drawn in the substantially center of the hand for gripping the control section.

17. The electrical bending endoscope according to Claim 1, wherein the error-operation preventing means comprises operation self-sensing means that allows an operator to sense his operation.

18. The electrical bending endoscope according to Claim 17, wherein the operation self-sensing means comprises force-sense changing means that allows the operation to be sensed by providing the inconstant amount of change in force.

19. The electrical bending endoscope according to Claim 17, wherein the operation self-sensing means comprises notifying means that notifies the operator of his operation.

20. The electrical bending endoscope according to Claim 19, wherein the notifying means generates sound to notify the operator.

21. The electrical bending endoscope according to Claim 19, wherein the notifying means generates light to notify the operator.

22. The electrical bending endoscope according to Claim 21, wherein the notifying means generates the light for notification in the field-of-view for observation of the endoscope.

23. The electrical bending endoscope according to Claim 21, further comprising,
a display device that displays observed images of an examinee,
wherein the notifying means generates the light for notification on a display screen of the display device.

24. The electrical bending endoscope according to Claim 19, wherein the notifying means generates vibrations to notify the operator.

25. The electrical bending endoscope according to Claim 24, further comprising,
a driving control device that controls the driving means, wherein the notifying means sends a notification with vibrations by the sense of touch by periodically driving the driving means to generate vibrations by the driving control device.

26. The electrical bending endoscope according to Claim 17, wherein the operation self-sensing means comprises input means that needs two or more steps of continuous operation.

27. The electrical bending endoscope according to Claim 17, wherein the operation self-sensing means comprises input means that needs two or more simultaneous parallel operations.

28. The electrical bending endoscope according to Claim 17, wherein the operation self-sensing means is additional input means that is detachably coupled to the control section arranged to the electrical bending endoscope.

29. The electrical bending endoscope according to Claim 1, wherein the error-operation preventing means includes switching-operation protecting means that protects the switching operating means.

30. The electrical bending endoscope according to Claim 29, wherein the switching-operation protecting means includes embedded input means that is arranged so as not to externally project from the exterior surface of the control section arranged to the electrical bending endoscope.

31. The electrical bending endoscope according to Claim 1, wherein the switching operating means comprises load distributing means that distributes applied load.
